(19) Europäisches Patentamt

European Patent Office

Office européen des brevets

(11) **EP 1 658 878 A1**

(12) **EUROPEAN PATENT APPLICATION**

(43) Date of publication:
24.05.2006 Bulletin 2006/21

(51) Int Cl.:
*A61N 5/10* $^{(2006.01)}$

(21) Application number: 04105841.3

(22) Date of filing: 17.11.2004

(84) Designated Contracting States:
AT BE BG CH CY CZ DE DK EE ES FI FR GB GR
HU IE IS IT LI LU MC NL PL PT RO SE SI SK TR
Designated Extension States:
AL HR LT LV MK YU

(71) Applicant: The European Community, represented
by the
European Commission
1049 Brussels (BE)

(72) Inventors:
• **Daquino, Giuseppe Giovanni**
**73040 Morciano di Leuca (Lecce) (IT)**
• **Cerullo, Nicola**
**56122 Pisa (IT)**

(74) Representative: **Ocvirk, Philippe et al**
**Office Ernest T. Freylinger S.A.**
**P.O. Box 48**
**8001 Strassen (LU)**

(54) **BNCT treatment planning**

(57) A method of computing the dose delivered to a target region by Boron Neutron Capture Therapy, wherein a target region of a patient is analysed to determine anatomical information as well as the actual *in vivo* distribution of the boron delivery agent, and wherein the dose delivered by irradiation within every cell of the target region is computed on the basis of said information on the target region's anatomy and on said actual *in vivo* distribution of the boron delivery agent.

Fig.1

**Description**

TECHNICAL FIELD

The present invention generally relates to cancer treatment by Boron Neutron Capture Therapy, and more specifically to treatment planning in BNCT.

BACKGROUND ART

Soon after the discovery of the neutron by Chadwick (1932), in 1936 Locher proposed the general concept of Neutron Capture Therapy (NCT) for cancer and his idea is still at the basis of current research in this field. NCT is a "binary" kind of therapy, in that treatment is carried out in two phases:

- *phase I:* the patient is infused with a drug whose molecule is designed to selectively concentrate in cancerous cells and contains an isotope having the peculiar nuclear property of a high probability of capturing neutrons;

- *phase II:* the patient undergoes irradiation with a beam of neutrons of suitable energy spectrum.

Since neutrons are electrically neutral, irradiation should result in little damage to healthy tissue, where the drug should ideally be absent, specially if "thermal" (i.e. very "slow") neutrons are employed.

$^{10}$B was soon identified as a suitable isotope for NCT: when interacting with a thermal neutron, the nucleus of this isotope has a very high probability of capturing the particle. This event soon causes the disintegration of the nucleus, which splits into an alpha particle and a recoiling $^7$Li nucleus. The two heavy charged particles have a combined energy of 2.33 MeV and an average range in tissue of 12-13 $\mu$m, which is the dimension of an average cellular diameter. As a result, the radiation damage due to the high LET ("Linear Energy Transfer") of the ions would ideally be very selectively delivered only to cancerous cells.

The first patient trials of boron-based NCT (BNCT) date back to the early 1950s and although the early results were not particularly encouraging, research went on through the years all around the world. Both neutron beam design and drug synthesis and delivery techniques have made dramatic advances meanwhile and currently patient trials are ongoing in The Netherlands (EORTC trials), Sweden, Finland, Italy, Czech Republic, USA, Japan and Argentina.

In all radiation therapy methodologies, great relevance is given to obtaining an estimation of the effects of the treatment on both tumor and healthy tissue under different potential irradiation configurations, in an effort to optimize all the parameters (duration, relative position of beam and patient, number of fields, beam characteristics etc.) to assure that the dose to healthy tissues will be kept within the prescribed limits.

This is an issue of particular concern in NCT because neutrons produce many different nuclear reactions in human tissues, each with a different physical and biological dose.

In this process, Treatment Planning Systems (TPSs), i.e. computer programs that assist the clinicians by simulating the interaction of radiation with tissues, play an essential role.

Particularly, present standard TP for *glioblastoma multiforme* (GBM - a kind of brain tumor), used in all BNCT trials, is based on the reconstruction (aided by Computed Tomography and/or Magnetic Resonance Imaging images) of a 3D model of the patient head. This model, with the introduction of data on the boron distribution, is implemented in a computer program which then simulates the interaction of radiation with tissues, computing the spatial distribution of the dose delivered to each anatomical structure. Due to the particularly complex physics of neutron interactions with matter, these programs are all based on the Monte Carlo technique, which actually simulates the "history" of every single particle in the radiation beam, instead of solving a set of equations, a task that would not be solvable in a complex three-dimensional geometry like that of human anatomy.

Several research centers in the world have been involved in the development of TPSs and great efforts have been made in recent years to optimize them for BNCT requirements.

Although the programs differ in many respects, they all share the same approach as far as the information on the distribution of boron in tissues is concerned: for the purpose of dose computation, boron is assumed to be uniformly distributed in each of a small number of "regions" in which the patient's head is subdivided. This approach results in a small set of possible values of boron concentration (very often just three, including zero) assigned to the actually numerous different tissues that are present. Furthermore, these values are "indirectly" estimated by means of an "educated guess" based on blood and tissue sample analysis. This situation was dictated by the absence of other information on the real *in vivo* distribution of the boron in the patient head.

In 1997 a group of Japanese researchers were able to describe through Positron Emission Tomography (PET) the *in vivo* $^{10}$B distribution, labeling the boron compound (BPA) with $^{18}$F (a positron emitter) and developing a model to estimate the $^{10}$B concentration value from the counts associated with each voxel (elemental volume) by the PET tomographer [1]. Several research groups since then have been able to image the boron carrier distribution *in vivo* and all these studies clearly showed that this distribution is actually very irregular, therefore far from the model normally implemented in TPS simulations.

OBJECT OF THE INVENTION

The object of the present invention is to provide an improved method for performing treatment planning in BCNT. This object is achieved by a method as claimed in claim 1.

GENERAL DESCRIPTION OF THE INVENTION

In order to improve treatment planning in BNCT, the present invention proposes to use information indicative of the actual *in vivo* boron distribution for treatment planning. Accordingly, the present method proposes the estimation (or computation or simulation) of the dose delivered to a patient's target organ and its surrounding tissues (hereinafter referred to as "target region") by Boron neutron capture therapy on the basis of combined information (obtained by appropriate analysis) on both the target region's anatomy and *in vivo* boron distribution.

Compared to prior art methods based on homogeneous boron distribution, the present method takes into account the actual-and in fact heterogeneous-boron distribution in the target region, and thus allows a more realistic simulation of the irradiation, which permits improved treatment planning.

The method is preferably implemented to compute the dose delivered by irradiation with every cell (or elemental volume) of the target region. The anatomical information on the target region may be obtained by Computed Tomography (CT) or Magnetic Resonance Imaging. The information regarding boron distribution in the target region of the patient-to whom a predetermined amount of boron (generally combined with a carrier or delivery agent) has been previously administered in view of BNCT-is preferably obtained by Positron Emission Tomography.

In a preferred embodiment, the method comprises:

- using diagnostic scanning means (preferably Computed Tomography (CT) or Magnetic Resonance Imaging (MRI) scanner) to scan the target region to produce cross-sectional images from which a three-dimensional model (hereinafter referred to as "3D model") of the target region is then generated;

- using Positron Emission Tomography (PET) to produce cross-sectional functional images from which a three-dimensional quantitative map (hereinafter referred to as "B model") of the actual boron carrier mass concentration in tissues is generated;

- co-registering the anatomical and the functional image stacks to build a model of the target region that is a three-dimensional array of three-dimensional cells, each incorporating at the location in space of every cell both the anatomical and the functional information (i.e. the boron carrier concentration in said location within the model);

- employing said model (hereinafter referred to as "MC model") to perform radiation transport simulations based on the Monte Carlo method to estimate (compute) the dose delivered by the irradiation within every cell.

The quantitative information about the concentration of the boron carrier in tissues is preferably derived from a stack of PET images.

In a preferred embodiment, the information derived from the PET images preferably pertains to the activity of a marker in the boron carrier (or delivery agent), such as the $^{18}F$ nuclei in the L-$^{18}F$-$^{10}B$-FBPA molecule, as detected and *measured in vivo* by the PET tomographer at the location of every single pixel of a PET image (corresponding to a voxel in the scanned region of space). The PET-derived information on the $^{18}F$ activity is advantageously *measured in vivo* at the location of every single voxel in every image is converted into a value of $^{10}B$ mass concentration by means of a suitable algorithm. The "suitable algorithm" for converting the $^{18}F$ activity into $^{10}B$ mass concentration can be based either on a "compartmental model" (as explained hereinbelow) or on a "normalization to the maximum activity value" model (as explained hereinbelow).

One important idea underlying the present invention was thus to link the PET information, related to the *in vivo* boron distribution, to the 3D modeling for the Monte Carlo simulation. Eventually, this idea led to the development of an original and simplified system, that created automatically an input file for the MCNP radiation transport code [2], including the real, three-dimensional map of boron distribution as given by the PET images.

The inventor's studies proved the feasibility of the present method and, as a natural consequence of that research, it was decided to create a complete TPS (now named BDTPS, i.e. "Boron Distribution Treatment Planning System"), that would allow the migration of this methodology to clinical BNCT trials.

Accordingly, the present invention also relates to a computer program comprising computer program code means adapted to perform the present method when run on a computer. It further relates to a computer program product comprising program code means stored on a computer readable medium for performing the present method, when run on a computer.

BDTPS is a software package (i.e. computer program and computer program product) in accordance with the invention whose task is to simulate the BNCT irradiation of the patient's organ, assuming a given set of positioning and beam parameters, and to compute the three-dimensional spatial distribution of the dose delivered to tumor and healthy tissue. Preferably, the procedure through which this is accomplished includes the following steps:

- acquisition of a three-dimensional data set describing the anatomy of the region to be irradiated, preferably by Computed Tomography (CT) or Magnetic Resonance Imaging (MRI);

- processing of the aforementioned data set to automatically build a "3D (geometric) model" of the region's anatomy. In this model the user defines different "regions", i.e. volumes identifying anatomical structures (e.g. target, organs at risk etc.) which are of special interest in the assessment of the irradiation efficacy. In this step a description of the materials each structure is made of (mass density and nuclear composition) is also given.

- acquisition of a three-dimensional data set describing the *in vivo* spatial distribution of the boron carrier in the region to be irradiated (based on PET analysis).

- processing of the aforementioned data set to automatically build a "boron model" (or "B model"), which will be used to compute the dose at a later stage.

- processing of all the above mentioned data structures to define a "Monte Carlo model", i.e. a description (written in the MCNP syntax) of all the relevant information that the radiation transport code will need in order to perform the simulation. This model, in analogy with the medical imaging data sets it is derived from, describes the region of space physically containing the patient's organs as a three-dimensional matrix of cells ("volume elements", or "voxels"), each filled with a given material.

- simulation of the irradiation and computation of the dose delivered to each cell on the basis of the real spatial distribution of the boron-carrier, stored in the "boron model" described above.

- display of the spatial distribution of the computed dose values as two dimensional maps (on the plane corresponding to each CT/MRI slice) or one-dimensional profiles along a line of arbitrary orientation, but parallel to the CR/MRI scanning planes.

The main advantage of the present invention, compared to all the other state-of-the-art TPSs is its more accurate description of the spatial distribution of the computed dose values. This is an essential improvement in accuracy and reliability of the treatment plan, because the final decision on which irradiation scheme, among those simulated, is to be preferred is made on the basis of the values of some figures of merit that have been proven to be dramatically influenced by the assumptions made on the distribution of boron nuclei in tissues [3].

In a preferred embodiment, the computer program product comprises program code means for performing the following steps:

- reading the files corresponding to two stacks of cross sectional images of the target region, one said stack providing the anatomical description of said target region, the other one said stack providing information on the *in vivo* boron distribution (e.g. by way of $^{18}$F activity) as detected by a PET tomographer, said files being preferably provided in QSH format, or said stack being converted to QSH prior to reading, if said stack is provided in another format such as e.g. RAW or 8-bit DICOM format;

- displaying said images on a computer display;

- allowing the user to define a set of two-dimensional "regions" (i.e. areas on the image within physical contours containing anatomical structures which can be considered "homogeneous" for the purposes of the user) on each anatomical image;

- creating a geometric model (3D model) of the target region in the form of a three-dimensional array of three-dimensional cells and displaying its three-dimensional representation;

- allowing interaction between the user and said representation to allow the definition of the "organs at risk", of the position of fiducial markers and of the radiation beam entry and exit point;

- creating a three-dimensional quantitative map (B model) of the boron carrier mass concentration in tissues on the basis of a stack of Positron Emission Tomography (PET) digital images;

- building an input file for the MCNP/MCNPX code (preferably) which is used by said code to perform the radiation transport simulation and includes both the anatomical information stored in the 3D model and the boron-distribution information stored in the B model;

- reading the MCNP output file and producing, according to the user's requests, a graphical representation of the results in the form of either one-dimensional profiles or 2D maps (on any CT/MRI image plane), and a table with computed dose data pertaining to all the organs at risk

BRIEF DESCRIPTION OF THE DRAWINGS

The present invention will now be described, by way of example, with reference to the accompanying drawings, in which:

FIG.1: BDTPS Flow Chart - Time (and logical) succession of the main steps in the BDTPS procedure;

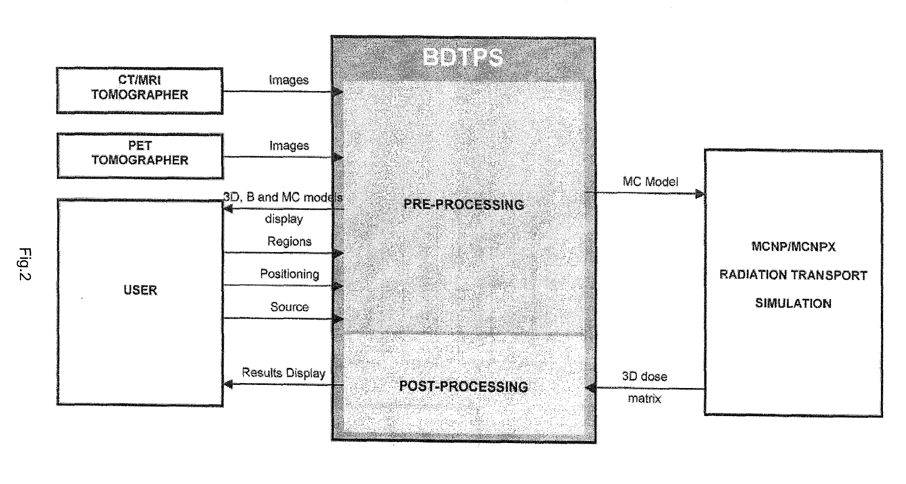

FIG.2: BDTPS Block diagram - Relationships between the different components of the BDTPS scheme;

FIG.3: Detailed flow chart of the sequence of steps in the BDTPS scheme;

FIG.4: HEBOM demounted (a) and mounted (b); and

FIG.5: Thermal neutron fluence rate, calculated by SERA, MCNP and BDTPS (experimental data are also reported).

DETAILED DESCRIPTION OF PREFERRED EMBODIMENTS

A preferred embodiment of the present method and more particularly its implementation as the BDTPS computer program will now be described in detail herebelow.

*Structure and operation*

Models building

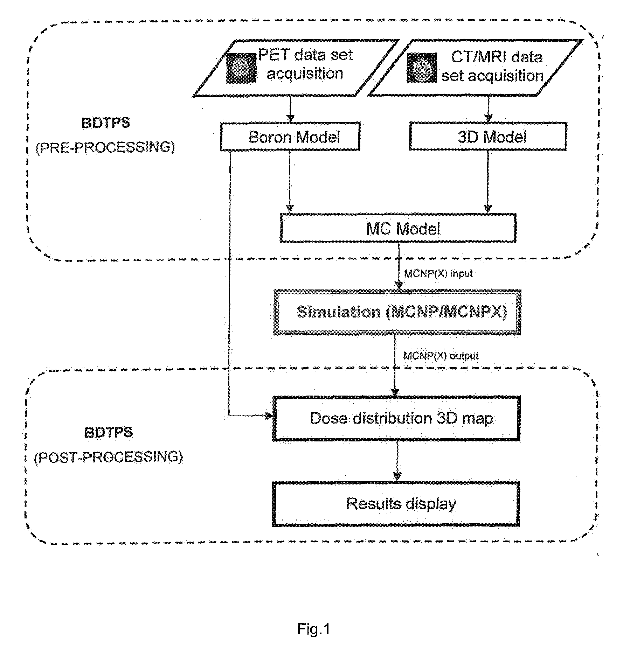

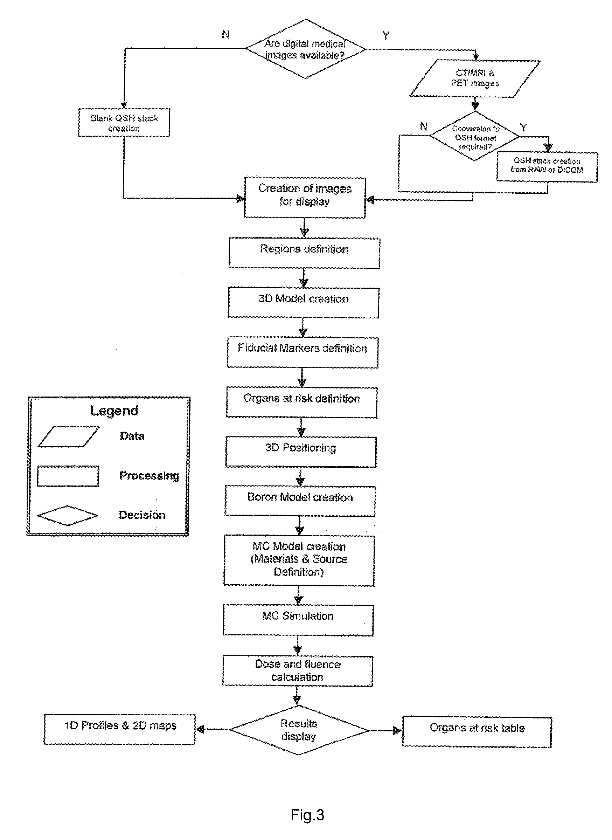

Except for the Boron Model, the BDTPS architecture (shown in Fig.1 in its essential traits and in greater detail in Fig.3) is quite similar to a standard BNCT TPS structure. In the pre-processing phase, the user constructs a 3D Model using the CT (or MRI) images as templates for depicting macro-regions (brain, target, skin, etc.). The macro-regions are created by a "stroke" tool, through which the user can define several control points on the CT images.
The depicted regions serve as a contour indication for the BDTPS algorithm, which transforms the included area into a matrix of the corresponding pixel values. Special care is taken in this phase in order to avoid errors for rounding particularly sharp contour edges.
Stacking together all the CT slices, the areas define a region volume, which can be represented through the BDTPS 3D engine ("3D World"). This environment also allows the user, after definition of the fiducial markers, to define the positioning of the patient with respect to the beam port. The fiducial markers are very important for the proper positioning of the patient, as these points constitute absolute points and allow to associate the coordinate of a positioning frame to that of the TPS system, in turn based on the CT system. An option available in BDTPS 3D World permits also to calculate the beam entrance and exit points inside this coordinate system, so that, using a proper program, it is possible to calculate the projections of these points onto the positioning frame. These projections constitute the reference for the positioning of the patient mask.
The 3D model is generally composed of less than one million of cells, each one associated with a specific material.
All these operations are performed in the *pre-processing* phase of BDTPS. This name indicates that it precedes the Monte Carlo calculations. In the next phase the Monte Carlo Model (MC Model) is built: BDTPS supports both the "mesh tally" format and the "standard lattice" format, allowing the user to freely choose whether to use MCNP or MCNPX, respectively. The "MCNP family" codes have constantly consolidated through the years their position as a standard de *facto* in radiation transport calculations, with applications including (but not limited to) design of accelerator spallation targets, investigations for accelerator isotope production and destruction, investigations of targets for accelerator-driven energy sources, medical physics (particularly proton and neutron therapy), investigations of radiation backgrounds and shielding for high altitude aircraft and spacecraft, accelerator-based imaging technology (particularly neutron and proton radiography), design of shielding in accelerator facilities, activation of accelerator components and surrounding groundwater and air, investigation of fully coupled neutron-charged particle transport for lower-energy applications, high-energy dosimetry and neutron detection, design of neutron experiments, comparison of physics-based and table-based data, charged-particle tracking in plasmas, charged-particle propulsion concepts for space flight, single-event upset in semiconductors, detection technology using charged particle.

This list accounts for the great versatility and reliability of these codes, which therefore represented a natural choice also for the BDTPS Monte Carlo engine.

**Construction of the Boron Model**

It is to be appreciated that the Boron Model constitutes an important feature of the invention; its construction and function are therefore described below in detail.

During the MC model construction, the Boron model (B Model) is also built, on the basis of the stack of PET images. BDTPS calculates the boron distribution in each PET slice and records the results in particular binary files (.bor files). The conversion of the PET counts recorded in the images into boron concentration values can be performed using two different techniques:

> i) using the *compartmental model,* fully described in [1] and [4]. This model can be described in a mathematical form that easily lends itself to implementation in a TPS.

When L-$^{18}$F-$^{10}$B-FBPA is administered i.v. to a human subject, the percentage of the dose circulating in the blood stream in usable form is important for the estimation of the tumour $^{10}$B level. If the time of the measurement is given, the percentage of the dose appearing in the arterial blood within this period can be determined. This percentage in the circulation of the total dose is defined as the *utilization ratio.* The utilization ratio of L-$^{18}$F-$^{10}$B-FBPA can be calculated by integration of the radioactivity appearing in the arterial blood relative to the total injection dose. This parameter is expressed as Ur*(t) and is a function of time. The utilization ratio is a factor representing the metabolism and excretion of L-$^{18}$F-$^{10}$B-FBPA.

The radioactivity Ci*(t) indicates the amount of incorporated L-$^{18}$F-$^{10}$B-FBPA in the tumour tissue. Dividing Ci*(t) by the radioactivity of L-$^{18}$F-$^{10}$B-FBPA in non-metabolised plasma Cp*(t) integrated over time t during the experiment, the incorporation ratio Ic*(t) is obtained. Ic*(t) is a function of time, but becomes a constant after a sufficient amount of time. In particular, it has been found that Ic*(t) tends to become constant after 42 minutes from the infusion in both the tumour and the normal tissue.

Furthermore, the metabolic fractions seem to be negligible during the 42 minutes after injection, because more than 95% of the total radioactivity in plasma is due to the free L-$^{18}$F-$^{10}$B-FBPA fraction during 42-min arterial sampling. Therefore, metabolic corrections are not necessary and L-$^{18}$F-$^{10}$B-FBPA metabolism can be treated as that of L-$^{10}$B-BPA.

Furthermore, the plasma $^{10}$B levels maintain linearity with the amount of L-$^{10}$B-BPA administered.

Making use of these parameters, it is possible to evaluate the $^{10}$B concentration from the PET data, taking into account the ratio between the L-$^{18}$F-$^{10}$B-FBPA and $^{10}$B weights:

$$^{10}B \ concentration \ (\mu g/kg) = total \ injection \ dose \ (\mu g) \ \cdot Ur^*(s/kg) \ \cdot Ic^*(s-1) \ \cdot$$

$$10/208$$

Therefore, PET studies using L-$^{18}$F-$^{10}$B-FBPA not only provide images of treatable brain tumours but also permit the determination of local $^{10}$B levels. This method is based solely on PET and can be used for real time pharmacokinetic and biodistribution studies.

> ii) Using the *normalization to the maximum activity value* recorded in each PET slice (for phantom applications, where the boron concentrations are known *a priori).* The PET machine can calculate a voxel-average activity in a region of interest, where the maximum activity in the slice is located. This activity is referred to as $\mu$Ci/cc. Because the real activity in that region is known in the phantom, the ratio between these two activity values can be easily calculated, yielding a scale factor that takes into account possible discrepancies, due to the calibration method used for the PET machine. Another scale factor is the ratio between the therapeutic boron concentration and the PET-measured boron concentration. The multiplication of these two scale factors gives the value required by BDTPS and called *Scaled Irradiation Factor (SIF)*.

In the normalization method, there is no need to take into account the metabolism effects related to the boron carrier. This is why neither the incorporation factor nor the utilization ratio are applicable to the phantom case. The final equation to calculate the boron concentration in each PET pixel is:

*B conc.(ppm) = (activity in the pixel in μCi/cc) · SIF · (μCi_to_μg factor) · (1/ρtissue)* where *μCi_to_μg factor* is the factor to translate μCi to μg and ρ*tissue* is the tissue density (in g/cc).

Since the B Model (at least in the second method) needs the material density associated to each PET pixel, the PET images must be coupled and co-registered with the MC model (and, therefore, with the 3D Model), containing the density information. Besides, in the post-processing phase the boron dose is computed by first dividing the normalized output value (note that MCNP calculates the tallies per unit of started particles) for each pixel by its region-averaged boron concentration and, afterwards, multiplying the result by the real boron concentration, associated to that pixel in the PET stack. Therefore, B Model and MC Model are strictly correlated to each other and prepared in the pre-processing phase. Since these two models are also correlated to the 3D original model, they actually make up a single logical structure, which constitutes an important aspect of this invention, which therefore offers the only integrated package for evaluating the BNCT planning using the real heterogeneous boron distribution.

Although BDTPS is designed to handle highly heterogeneous boron concentration spatial distributions, in case of lacking *in vivo* boron distribution data it is possible to assign manually boron concentration values to macro-zones depicted on the CT images, following the current standard approach to BNCT TP.

Radiation transport simulation and dose calculation

The default BDTPS calculations are referred to the main doses and fluences to take into account in BNCT:

- neutron fluence (thermal, epithermal, fast and total);

- photon fluence;

- boron dose;

- hydrogen neutron capture dose (or gamma dose);

- proton recoil dose;

- nitrogen dose.

The photon fluence and dose can be calculated both with and without the photon source contribution. The nitrogen dose is always calculated, due to the presence of air all around the 3D model voxels.

The kerma factors used for the photon dose calculations are reported in ICRU-46 [5], while the kerma factors used for the proton recoil and nitrogen doses are taken from ICRU-63 [6]. Finally, the boron dose is calculated through the kerma factors reported in [7].

Also two other composite doses can be calculated:

- total physical dose (without any weighting factor);

- total weighted dose (using the RBE factors, mainly adopted in BNCT).

The default RBE factors present in BDTPS are:

- $^{10}$B reaction dose: 2.3

- Proton recoil reaction dose: 3.2

- Nitrogen reaction dose: 3.2

- Gamma reaction dose: 1.0

**Display of results**

Once the Monte Carlo engine (MCNPX or MCNP) has completed the required calculations, the *post-processing phase* begins. BDTPS can display the results (doses and fluences) as 2D maps (on any CT/MRI image plane) or mono-dimensional profile along an arbitrary direction belonging to any CT/ MRI image plane. The latter is permitted by the line edit tool, which actually calculates the required values, using a tracking algorithm into the values matrix along the user-defmed line. The present version of BDTPS can apply this algorithm on lines parallel to the CT planes.

Furthermore, a table with computed dose data pertaining to all the organs at risk can be displayed.

Figure 1 shows the flow diagram for BDTPS, containing the main characteristics of this treatment planning system. More detailed explanations on the present method and its computerized implementation can be found in the inventor's document [3], which is incorporated herein by reference.

**BDTPS experimental and computational validation**

The validation of the PET-based treatment planning system requires the use of phantoms, which should be able to reproduce as much as possible the heterogeneous boron distribution. In this way, the validation exercise can be considered as close as possible to the real situation, which appears from the PET scanning.

On the other hand, the experimental requirements impose the monitoring of the main parameters during the irradiation, which in turn limits somehow freedom in the choice of the phantom geometry. For example, the necessity to have inter-spaces for the insertion of detectors prevents from disposing different boron concentrations very close to each other.

The measurements have been performed at the Joint Research Centre (JRC) located in Petten (The Netherlands), where the 45MW HFR nuclear reactor is present.

Since none of the phantoms available at JRC-Petten met the specific requirements of this validation study, a new phantom named HEBOM (HEeterogeneous BOron phantoM) was designed and built [3].

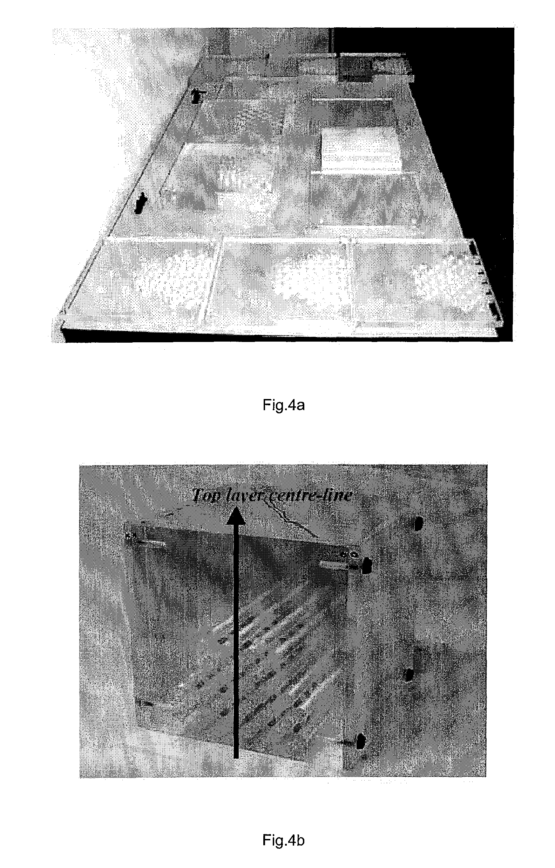

HEBOM's final design (see Fig. 4) allows up to 64 vials to be placed in four layers, containing 8 different boron concentrations. Each vial has different colour depending on its boron content. The decision to colour the vials has been taken in order to facilitate a quick vial recognition during the PET loading phase. The vials slabs are located in the centre of the phantom.

All the slabs contain clearance holes (15 mm diameter), between the vials, to host neutron and gamma detectors.

To provide the information necessary to reconstruct the 3D and the Boron Models in BDTPS, HEBOM had to be scanned with a CT and a PET machine.

The strict collaboration with Istituto di Fisiologia Clinica (CNR), located in Pisa, allowed for the PET scanning, while the CT scanning has been performed at the Department of Radio-diagnostics (S. Chiara Hospital in Pisa). The HEBOM CT images served for the *3D model* reconstruction, while the PET images constituted the basis for the *B model.*

HEBOM has been irradiated at HB11 Petten and the main parameters (neutron flux, gamma, fast and neutron dose) have been monitored using the standard techniques utilized also in the BNCT clinical routine. The positioning of HEBOM has been performed using the standard laser technique, also adopted for the patient's head positioning. P/N diodes were used for measuring the thermal neutron fluence, while paired Ar/Mg and TE/TE ionization chambers measured the fast neutron fluence. The Ar/Mg chambers were used also for the gamma dose evaluation. The BDTPS experimental validation, performed through the HEBOM measurement campaign, was integrated with computational tests made using both SERA and MCNP-4C3.

SERA was used as reference treatment planning system, because it is pixel-based like BDTPS. However, due to the uncertainties related to the application of a standard TPS to the heterogeneous boron phantom, a confirmation of the results was checked through an analytical model, using MCNP-4C3.

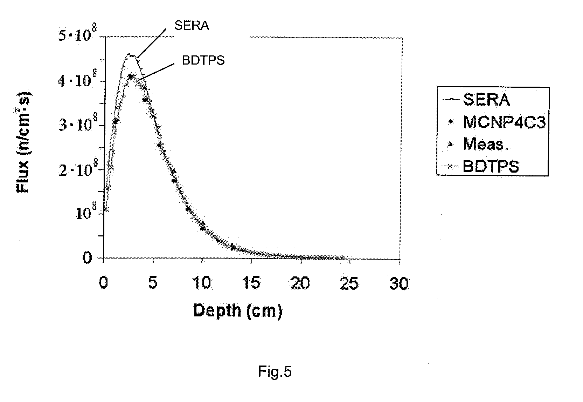

Complete agreement was found between the results of BDTPS and the analytical model in all HEBOM layers and for all the monitored parameters, such as the boron dose, the thermal neutron flux (Fig. 5), the proton recoil dose and the total gamma dose. Fig. 5 shows the difficulty for SERA to estimate the thermal neutron fluence, especially on the border between air and the first face of the phantom.

**_References_**

[1] Y. Imahori, S. Ueda, Y. Ohmori, T. Kusuki, K. Ono, R. Fujii, T. Ido - Fluorine-18-labelled Fluoroboronophenylalanine PET in patients with glioma, *J. Nud. Med.,* 1998, 39(2): 325-333

[2] J.F. Briesmeister, MCNP - A general Monte Carlo N-particle transport code Version 4B, LA12625-M, Los Alamos National Laboratory, 1997

[3] G.G. Daquino - A PET-based Treatment Planning System to evaluate the influence of the heterogeneous boron distribution in Boron Neutron Capture Therapy, Ph.D. Thesis, Università di Pisa and Joint Research Centre of the European Commission, Institute for Energy. *Not published yet*

[4] Imahori Y., Ueda S., Ohmori Y., Sakae K., Kusuki T., Kobayashi T., Takagaki M., Ono K., Ido T. and Fujii R. - Positron Emission Tomography-based Boron Neutron Capture Therapy Using Boronophenylalanine for High-Grade Gliomas: Part I, *Clinical Cancer Research* 1998; 4:1825-1832

[5] ICRU Report 46 - "Photon, Electron, Proton and Neutron Interaction Data for Body Tissues", International Commission on Radiation Units and Measurements, 1992

[6] ICRU Report 63 - "Nuclear Data for Neutron and Proton Radiotherapy and for Radiation Protection", International Commission on Radiation Units and Measurements, 2000

[7] Caswell R.S., Coyne J.J., Randolf M.K. - Kerma factors of elements and compounds for neutron energies below 30 MeV, *Int. J. Appl. Radiat. Isot.,* 1982; 33:1227-1262

## Claims

1. A method of estimating the dose delivered to a target region by Boron Neutron Capture Therapy, comprising the steps of:

   analysing a target region of a patient to determine anatomical information on said target region; and
   computing the dose delivered by irradiation within said target region based on said anatomical information and on boron distribution values in said target region;
   **characterized in that**
   said target region is also subjected to analysis to determine information on the *in vivo* boron distribution; and said boron distribution values used for said computation of the dose delivered by irradiation are derived from said information on the *in vivo* boron distribution.

2. The method according to claim 1, comprising:

   - using diagnostic scanning means to scan the target region to produce anatomical cross-sectional images from which a three-dimensional model (3D model) of the target region is then generated;
   - using Positron Emission Tomography (PET) to produce cross-sectional functional images from which a three-dimensional quantitative map (B model) of the boron carrier mass concentration in tissues is generated;
   - co-registering the anatomical and the functional images to build a model of the target region that is a three-dimensional array of three-dimensional cells, each incorporating at the location in space of every cell both the anatomical and the functional information;
   - employing said model (MC model) to perform radiation transport simulations based on the Monte Carlo method to compute the dose delivered by the irradiation within every cell.

3. The method according to claim 2 wherein the quantitative information about the concentration of the boron carrier in tissues is derived from a stack of PET images.

4. The method according to claim 3 wherein the information derived from the PET images pertains to the activity of a marker combined to the boron carrier as detected and *measured in vivo* by the PET tomographer at the location of every single pixel of a PET image.

5. The method according to claim 3 wherein the PET-derived information on the marker activity *measured in vivo* at the location of every single voxel in every image is converted into a value of $^{10}$B mass concentration by means of a suitable algorithm.

6. The method according to claim 5 wherein said suitable algorithm for converting the marker activity into $^{10}$B mass concentration can be based either on a compartmental model or on a normalization to the maximum activity value model.

7. The method according to claim 2 wherein the MC model incorporates both a geometric description of the target region's anatomy and a description of the real *in vivo* spatial distribution of the $^{10}$B mass concentration values within said geometric description.

8. The method according to claim 7 wherein the value of mass concentration of $^{10}$B to be assigned to each cell is determined by its position within the geometric model of the target region.

9. The method according to claim 8 wherein the $^{10}$B mass concentration value is assigned to a position within the geometric model by establishing a correspondence between the spatial coordinates of the 3D model and those of the B model.

10. A computer program comprising computer program code means adapted to perform the method as defined in any one of the preceding claims when said program is run on a computer.

11. A computer program product comprising program code means stored on a computer readable medium for performing the method as defined in any one of claims 1 to 9 when said program product is run on a computer.

12. A computer program product for assisting clinicians in the development of a treatment plan for Boron Neutron Capture Therapy, said computer program product comprising program code means stored on a computer readable medium for, when run on a computer, performing the steps of:

> - reading the files corresponding to two stacks of cross sectional images of the target region, one said stack providing the anatomical description of said target region, the other one said stack providing information on the *in vivo* boron distribution as detected by a PET tomographer;
> - displaying said images on a computer display;
> - allowing the user to define a set of two-dimensional "regions" on each anatomical image;
> - creating a geometric model (3D model) of the target region in the form of a three-dimensional array of three-dimensional cells and displaying its three-dimensional representation;
> - allowing interaction between the user and said representation to allow the definition of organs at risk, of the position of fiducial markers and of the radiation beam entry and exit point;
> - creating a three-dimensional quantitative map (B model) of the boron carrier mass concentration in tissues on the basis of a stack of Positron Emission Tomography (PET) digital images;
> - building an input file to perform the radiation transport simulation, said imput file including both the anatomical information stored in the 3D model and the boron-distribution information stored in the B model;
> - reading the output file resulting from said simulation and producing, according to the user's requests, a graphical representation of the results in the form of either one-dimensional profiles or 2D maps, and a table with computed dose data pertaining to all the organs at risk.

13. A method of simulating the dose delivered to a target region by Boron Neutron Capture Therapy on the basis of combined information on both the target region's anatomy and the *in vivo* distribution of the boron delivery agent, said method comprising:

> using a diagnostic Computed Tomography (CT) or Magnetic Resonance Imaging (MRI) scanner to scan the target region to produce cross-sectional images from which a three-dimensional model (3D model) of the target region is then generated;
> using Positron Emission Tomography (PET) to produce cross-sectional functional images from which a three-dimensional quantitative map (B model) of the boron carrier mass concentration in tissues is generated;
> co-registering the anatomical and the functional image stacks to build a model of the target region that is a three-dimensional array of three-dimensional cells, each incorporating at the location in space of every cell both the anatomical and the functional information;
> employing said model (MC model) to perform radiation transport simulations based on the Monte Carlo method to compute the dose delivered by the irradiation within every cell.

Fig.1

Fig.2

Fig.3

Fig.4a

Fig.4b

Fig.5

**European Patent Office**

## PARTIAL EUROPEAN SEARCH REPORT

which under Rule 45 of the European Patent Convention shall be considered, for the purposes of subsequent proceedings, as the European search report

Application Number

EP 04 10 5841

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | GEORGE W. KABALKA ET AL: "Evaluation of Fluorine-18-BPA-Fructose for Boron Neutron Capture Treatment Planning" THE JOURNAL OF NUCLEAR MEDICINE, vol. 38, no. 11, 1997, pages 1762-1767, XP009047186 * abstract * * page 1762, right-hand column, paragraphs 1,2 * * page 1763, right-hand column, line 40 - line 60 * * page 1764, left-hand column, line 30 - line 40 * * page 1764, right-hand column, line 40 - line 60 * * page 1765, right-hand column, line 30 - line 40 * * page 1766, left-hand column, line 1 * * page 1766, right-hand column, line 30 - line 40 * ----- -/-- | 10-12 | A61N5/10 |

TECHNICAL FIELDS SEARCHED (Int.Cl.7)

A61N

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 4 May 2005 | Rodríguez Cossío, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent
Office

**PARTIAL EUROPEAN SEARCH REPORT**

Application Number

EP 04 10 5841

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | IAEA: "Current Status of neutron capture therapy" May 2001 (2001-05), INTERNATIONAL ATOMIC ENERGY AGENCY , VIENNA , XP002326807 | 10-12 | |
| A | * page 38 - page 45 * | 10-12 | |
| A | * page 175 - page 185 * | 10-12 | |
| A | * page 206 - page 215 * | 10-12 | |
| A | * page 216 - page 222 * | 10-12 | |
| X | * page 223 - page 232 * * page 224, line 1 - line 10 * ----- | 10-12 | |
| A | NAKAGAWA Y. AND HATANAKA H.: "Boron neutron capture therapy" JOURNAL OF NEURO-ONCOLOGY, vol. 33, 1997, pages 105-115, XP002326805 THE NETHERLANDS * page 108, left-hand column, lines 1,2 * ----- | | **TECHNICAL FIELDS SEARCHED** (Int.Cl.7) |
| Y | US 5 341 292 A (ZAMENHOF ET AL) 23 August 1994 (1994-08-23) * column 6, line 15 - line 34 * * abstract * ----- | 10-12 | |
| Y | US 2003/155530 A1 (ADNANI NABIL ET AL) 21 August 2003 (2003-08-21) * paragraph [0148] - paragraph [0150] * ----- | 10-12 | |
| A | US 6 049 729 A (COOK ET AL) 11 April 2000 (2000-04-11) * the whole document * ----- | 12 | |
| A | US 5 995 864 A (WESSOL ET AL) 30 November 1999 (1999-11-30) * the whole document * ----- | 12 | |

-/--

EPO FORM 1503 03.82 (P04C10)

# EP 1 658 878 A1

**European Patent Office**

## PARTIAL EUROPEAN SEARCH REPORT

Application Number

EP 04 10 5841

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| A | KOBAYASHI T. AND SAKURAI Y.: "A nonnvasive dose estimation system for clinical BNCT based on PG-SPECT" MED. PHYS., vol. 27, no. 9, September 2000 (2000-09), pages 2124-2132, XP002326806 * abstract * ----- | 10-12 | |
| A,D | IMAHORI ET AL: "Positron emission tomography-based Boron Neutron Capture Therapy Using Boronophenylanine for High-Grade Gliomas: Part I" CLINICAL CANCER RESEARCH, vol. 4, August 1998 (1998-08), pages 1825-1832, XP001205880 * the whole document * ----- | 10-12 | TECHNICAL FIELDS SEARCHED (Int.Cl.7) |
| A | IMAHORI ET AL: "Positron emission tomography-based Boron Neutron Capture Therapy Using Boronophenylanine for High-Grade Gliomas: Part II" CLINICAL CANCER RESEARCH, vol. 4, August 1998 (1998-08), pages 1833-1841, XP001206193 * the whole document * ----- | 10-12 | |
| A | GEORGE W. KABALKA ET AL: "In vivo Boron-11 MRI and MRS using (B24H22S2)4 in the rat" MAGNETIC RESONANCE IMAGING, vol. 9, 1991, pages 969-973, XP001206281 USA * page 969, left-hand column * ----- | 10-12 | |

EPO FORM 1503 03.82 (P04C10)

**European Patent Office**

**INCOMPLETE SEARCH SHEET C**

Application Number

EP 04 10 5841

Claim(s) not searched:
    1-9,13

Reason for the limitation of the search (non-patentable invention(s)):

Article 52 (4) EPC - Method for treatment of the human or animal body by surgery

**EP 1 658 878 A1**

**ANNEX TO THE EUROPEAN SEARCH REPORT
ON EUROPEAN PATENT APPLICATION NO.**      EP 04 10 5841

This annex lists the patent family members relating to the patent documents cited in the above-mentioned European search report.
The members are as contained in the European Patent Office EDP file on
The European Patent Office is in no way liable for these particulars which are merely given for the purpose of information.

04-05-2005

| Patent document cited in search report | | Publication date | Patent family member(s) | | Publication date |
|---|---|---|---|---|---|
| US 5341292 | A | 23-08-1994 | NONE | | |
| US 2003155530 | A1 | 21-08-2003 | AU<br>CA<br>WO | 2820501 A<br>2396928 A1<br>0151124 A2 | 24-07-2001<br>19-07-2001<br>19-07-2001 |
| US 6049729 | A | 11-04-2000 | AU<br>WO | 1198999 A<br>9920169 A2 | 10-05-1999<br>29-04-1999 |
| US 5995864 | A | 30-11-1999 | AU<br>WO | 9571598 A<br>9913772 A1 | 05-04-1999<br>25-03-1999 |

EPO FORM P0459

For more details about this annex : see Official Journal of the European Patent Office, No. 12/82